# EUROPEAN PATENT SPECIFICATION

(11) **EP 3 496 731 B1**
(45) Date of publication and mention of the grant of the patent: **14.06.2023**
(21) Application number: 17757570.1
(22) Date of filing: 27.07.2017
(51) Int. Cl.: A61K 35/20, A61K 31/7034, A61K 31/737, A61K 9/00

(54) **COMPOSITION FOR THE TREATMENT OF PATHOLOGIES OF THE URINARY SYSTEM**
ZUSAMMENSETZUNG ZUR BEHANDLUNG VON ERKRANKUNGEN DER HARNWEGE
COMPOSITION POUR LE TRAITEMENT DE PATHOLOGIES DU SYSTÈME URINAIRE

(30) Priority: 10.08.2016 IT 201600084488
(43) Date of publication of application: 19.06.2019
(73) Proprietor: Alfakjn S.r.l., 27026 Garlasco (PV) (IT)
(72) Inventor: FERRARI, Alessio, 27026 Garlasco (PV) (IT); GENOVA, Luciano, 27026 Garlasco (PV) (IT)
(74) Representative: Hoffmann Eitle
(86) International application number: PCT/IB2017/054558
(87) International publication number: WO 2018/029558

(56) References cited:
- WO-A1-2010/028652
- Anonymous: "Health and Beauty: BENEFITS OF COLOSTRUM", , 17 June 2012 (2012-06-17), XP055382843, Retrieved from the Internet: URL:http://gloriainc.blogspot.de/2012/06/d r.html [retrieved on 2017-06-19]
- S TUCCI: "Colostrum in menopause effects on vaginal cytology/symptoms.", CLIN EXP OBSTET GYNECOL., vol. 40, no. 2, 1 January 2013 (2013-01-01), pages 219-221, XP055382837,
- ORIANA CIANI ET AL: "Intravesical administration of combined hyaluronic acid (HA) and chondroitin sulfate (CS) for the treatment of female recurrent urinary tract infections: a European multicentre nested case-control study", BMJ OPEN, vol. 6, no. 3, 1 March 2016 (2016-03-01), page e009669, XP055383314, ISSN: 2044-6055, DOI: 10.1136/bmjopen-2015-009669

## Description

The present invention relates to a composition comprising a mixture that comprises or, alternatively, consists of an effective amount of colostrum and at least one pharmaceutical grade additive, said composition being for use in the treatment of pathologies of the urinary system. In particular, the present invention relates to said composition for use in the treatment of the chronic pelvic pain, in particular of cystitis and more preferably of interstitial cystitis, wherein said composition is administered by intravesical administration.

The present invention relates to a composition comprising a mixture which comprises or, alternatively, consists of the following components:
a) colostrum;
b) chondroitin sulphate, or the salts thereof, and, optionally,
c) salicin,
said composition further comprises at least one pharmaceutical or food grade additive, wherein said composition is administered by intravesical administration.

The present invention relates to a medical device comprising said composition.

It is known that pelvic pain, i.e. abdominal pain localised in the region below the navel, at the level of the lower abdomen, the pelvic floor and/or the genital area, is a common symptom that may be originated in pelvic or extrapelvic organs or may be secondary to a systemic disease. It is often not possible to define any cause.

"Symptom" means a manifestation of an organic or functional alteration which, in general, presents itself as a characteristic sign of a disease.

Acute pelvic pain can be due to a surgical emergency (e.g. torsion of an ovarian cyst, an ectopic pregnancy, rupture of a tubo-ovarian abscess, appendicitis, or an intestinal perforation).

"Chronic pelvic pain" means a constant or cyclical pain lasting for over six months, which is localised at the anatomical pelvis, and severe enough to cause functional disability that requires medical or surgical treatment.

Whereas acute pelvic pain is nearly always the symptom of a pathological condition, chronic pelvic pain can itself represent a pathology. For subjects who suffer from chronic pelvic pain, it is difficult to separate the organic component from the psychological one. Such subjects are usually anxious and depressed and their working, social and marital life is often compromised.

The urinary system is made up of the kidneys and the urinary tract, which is made up of the ureters, the bladder and the urethra.

Cystitis is an inflammation of the bladder and is commonly caused by a bacterial urinary tract infection.

Interstitial cystitis (IC), or bladder pain syndrome (BPS), is a chronic debilitating syndrome characterised by recurrent abdominal and pelvic discomfort or pain in the absence of urinary tract infections. Its symptoms include discomfort, an increase in bladder pressure, sensitivity and intense pain in the vesical and pelvic regions, a greater urinary frequency and urgency, and often a combination of these symptoms. The pain often worsens during menstruation and may intensify during sexual intercourse. The symptoms also worsen with bladder filling and improve after bladder emptying.

For these reasons, interstitial cystitis is a pathology that has a very negative impact on the quality of life. This condition has a prevalence rate of 2.71% and 1.22% in women and men, respectively, even if these rates depend on the definitions used. It is believed that in Italy there are at least 10,000 people who suffer from IC, but this figure could be considerably underestimated due to difficulties in diagnosis. The etiology of IC is not yet well understood and various hypotheses are being investigated.

Interstitial cystitis (IC) is very different from common cystitis, which is a urinary tract infection caused by specific bacteria. IC, by contrast, is provoked by a cellular alteration of the bladder walls whose cause is still unknown. It is believed that a lesion or deterioration of the urinary epithelium has a fundamental role in the formation of IC. In particular, in the initial stage of IC, lesions of a multiple nature form on the internal epithelium, above all affecting the state of the glycosaminoglycans (GAGs) forming the bladder mucosa. The acids and possible toxins present in urine can thus come into contact with the nerve endings of bladder tissues and bring about an increase in the local nerve sensitivity that regulates pain in the affected area and increases the urge to urinate.

In some cases it is possible to establish a link between the onset of interstitial cystitis and localised pharmacological and/or radiotherapeutic treatments in the pelvic area, for example for cancer therapy on the bladder and adjacent regions.

In the majority of cases, however, the etiology remains uncertain and various hypotheses have been formulated, including autoimmune processes, allergic reactions, chronic bacterial infections, exposure to toxic substances or dietary components, psychosomatic factors and immunodeficiency, even slight.

The products currently available for IC are generally based on glycosaminoglycans (GAGs), such as chondroitin sulphate, or the salts thereof, hyaluronic acid and combinations thereof, in the presence of calcium ions. However, it has been found (Cervigni, M. Transl Androl Urol 2015, 4(6), 638 and Gülpinar, Ö. et al Can Urol Assoc J 2014, 8, E610-4) that these therapies have a response, in terms of improving the symptoms, in between approximately 30 and 70% of the subjects treated; there is thus a considerable number of subjects who suffer from interstitial cystitis and are resistant to treatment with the products presently on the market.

In the context of the present invention, "treatment" means an intervention comprising the administration of a substance, or mixture of substances, and having the aim of eliminating, reducing or preventing a pathology and its symptoms.

A plant-based formulation (oil of baobab, genus *Adansonia*) containing pectins, glucides, saponins, sterols, triterpenes and vitamin C has recently been made available as an intravesical adjuvant for patients with symptoms of cystitis induced by cancer immunotherapy with bacillus Calmette-Guerin (BCG), who do not respond to drugs such as anticholinergics, antibiotics, alpha-blockers and anti-inflammatory drugs, but the effectiveness of the formulation in subjects resistant to GAG-based therapies is not known.

It follows that there is still a felt need to have a composition that can be used for the curative or preventive treatment of symptoms or pathologies of the urinary system, in particular the symptoms of pelvic pain or acute pelvic pain, or the pathology of chronic pelvic pain, such as those caused by cystitis or interstitial cystitis, above all in subjects who are resistant to the currently available therapies based on glycosaminoglycans (GAGs) such as chondroitin sulphate and hyaluronic acid .

After a long, intense research and development activity, the inventors have provided an answer for the above-mentioned limits and drawbacks present in the prior art by devising a composition comprising a mixture which comprises or, alternatively, consists of an effective amount of colostrum, in natural, purified or at least partially purified form, said composition being for use in the curative or preventive treatment of symptoms or pathologies of the urinary system, preferably of the bladder.

The present invention further relates to a device suitable for intravesical administration, comprising at least one container which contains the composition comprising colostrum having the features defined in any one of the claims appended hereto.

Preferred embodiments of the present invention will be described here below in detail, there being no intention to limit the scope and significance of the present invention in any way.

Unless otherwise specified, the content of an ingredient in a composition relates to the percentage by weight of that ingredient relative to the total weight of the composition.

Unless otherwise specified, the indication that a composition "comprises" one or more components means that other components can be present in addition to the one or ones specifically named and the indication that a composition "consists" of given components means that the presence of other components is ruled out.

Within the scope of the present invention, "colostrum in natural form" (first milk) means a serous fluid having the complete composition with which it is secreted by the mammary glands during pregnancy and the first days after birth, i.e. without undergoing purification or any separation of components of the naturally produced fluid. Colostrum is composed mainly of water, leukocytes, proteins (including immunologic agents), fats and carbohydrates. The definition of colostrum is understood to include said composition in solid form, e.g. obtained by dehydration or separation of the aqueous portion from the other components.

In the context of the present invention, "colostrum" is understood to include colostrum in natural form, colostrum in at least partially purified form and/or colostrum in purified form.

Within the scope of the present invention, "colostrum in at least partially purified form" means a liquid, semi-solid or solid composition obtained from colostrum after separating at least one portion of a component thereof, such as, for example, casein, fats and/or lactose.

In the composition for use according to the present invention, the colostrum is preferably of non-human origin, more preferably of bovine, ovine or equine origin, even more preferably of bovine origin. The mixture and thus the composition according to the present invention comprises colostrum in purified or at least partially purified form, preferably colostrum of bovine origin.

The composition for use according to the present invention preferably comprises colostrum in purified form containing less than 2% by weight/total weight of the colostrum of at least one component among fats, immunogenic caseins or reducing sugars such as lactose; more preferably, said colostrum contains less than 1% or 0.1% by weight of each of said components.

The colostrum in the composition for use according to the present invention preferably has a protein content of at least 80%, preferably from 85% to 98% by weight relative to the total weight thereof. The colostrum in the composition for use according to the invention preferably contains at least one ingredient among immunoglobulins G, A and M, alpha lactalbumin, beta lactoglobulins, lactoferrin, transferrin, IGF-1, TGF, IFN-gamma, TNF, IL-2 and mixtures thereof. Immunoglobulin G preferably constitutes at least 75% by weight of the total weight of the colostrum in the composition for use according to the present invention. In a preferred embodiment of the invention, said composition comprising colostrum is for use in the curative or preventive treatment of the symptoms of pelvic pain or acute pelvic pain, or the pathology of chronic pelvic pain, particularly of cystitis, more preferably of interstitial cystitis.

Preferably, said composition is in liquid form and is for topical (intravesical) administration.

Said composition can be used for the treatment of male or female subjects.

In a preferred embodiment of the invention, said composition comprising colostrum is for use in the treatment of pelvic pain or acute pelvic pain or chronic pelvic pain in subjects who have undergone or are still undergoing radiotherapy or chemotherapy, more preferably for use in the treatment in said subjects of interstitial cystitis which may be induced by such therapies.

In a preferred embodiment of the invention, said composition comprising colostrum is for use in the treatment of cystitis, more preferably of interstitial cystitis, in patients resistant to other therapies for chronic pelvic pain, particularly to therapies comprising the administration of glycosaminoglycans.

Within the scope of the present invention, "resistant patient" means a subject in whom a previous therapy did not provide the expected effects after administration of the therapy for a specific amount of time, determined by the medical practitioner based on the type and severity of the pathology.

The composition according to the present invention has been found to advantageously reduce the symptoms of cystitis (inflammation of the bladder), particularly of interstitial cystitis (pelvic pain syndrome), in subjects who have previously undergone therapies based on GAGs (such as hyaluronic acid, chondroitin sulphate or combinations thereof) without the expected results.

In a preferred embodiment, the composition for use according to the invention comprises colostrum, in natural or purified or at least partially purified form, in an amount by weight comprised from 0.5 to 5%, more preferably 1 to 3%, 1.5 to 2.5% or 1.8 to 2%, relative to the total weight of the composition.

In a preferred embodiment of the present invention, the composition for use as described above comprises, in addition to colostrum as defined above, at least one other active ingredient selected from among chondroitin sulphate, or the salts thereof, such as the sodium salt, salicin and mixtures thereof.

It is known from the Merck Index (14th edition) that chondroitin sulphate is a generic term indicating a polymer having an average molecular weight of about 50,000 Da (atomic units). Chondroitin sulphate can be of animal origin or produced using biotechnological methods such as, by way of non-limiting example, by fermentation, as described in WO 2012/004063 A1.

Various forms of chondroitin sulphate exist, e.g. chondroitin 4-sulphate A [CAS No. 24967-93-9], the sodium salt thereof [CAS No. 9082-07-9] or the disodium salt thereof [CAS No. 39455-18-0].

The form chondroitin 6-sulphate (chondroitin sulphate C) also exists -CAS No. 25322-46-7.

In the context of the present invention, it is envisaged to use one of the above-mentioned forms.

The chondroitin sulphate (optionally in the form of sodium salt) in the composition for use according to the present invention preferably has an average molecular weight that depends on the number of disaccharide units, i.e. on the specific form of said chondroitin that is used, and it can be determined, for example, as described in Igarashi, N. et al Journal of Carbohydrate Chemistry, vol. 2013, Article ID 856142, 9 pages, or as described in WO 2012/159655 A1. Preferably, by way of non-limiting example, the chondroitin sulphate can have an average molecular weight of 1000 to 100000 Da, more preferably 2000 to 70000 Da, and even more preferably 5000 to 50000 or 10000 to 20000 Da.

"Salicin" or "D-salicin" means a beta-glucoside (IUPAC name (2*R*,3*S*,4*S*,5*R*,6*S*)-2-(Hydroxymethyl)-6-[2-(hydroxymethyl) phenoxy]oxane-3,4, 5-triol) having anti-inflammatory action and present mainly in willow bark. In a preferred embodiment, the composition for use according to the present invention comprises a plant extract (e.g. of willow bark) containing salicin in an amount of no less than 80%, more preferably no less than 90% and/or no more than 95%, preferably no more than 99% weight/total weight of the extract.

It has been found that by administering a mixture of colostrum, chondroitin sulphate and salicin, one advantageously obtains a restoration of the urothelial coating, a reduction in the inflammatory process and a reduction in pain, with full therapeutic completeness.

It is understood that, within the scope of the present invention, the active ingredients can all be administered simultaneously, i.e. via a mixture or, alternatively, they can be administered sequentially, i.e. separately from one another, in a time interval determined based on needs.

The present invention relates to a composition comprising a mixture which comprises or, alternatively, consists of the following components:
a) colostrum;
b) chondroitin sulphate, or the salts thereof, and, optionally,
c) salicin,
said composition further comprising at least one pharmaceutical grade additive and, optionally, at least one food grade additive.

In a preferred embodiment, in addition to colostrum, the composition for use according to the invention comprises chondroitin sulphate, or the salts thereof, and salicin, each independently of the other, in an amount by weight comprised from 0.5 to 5%, more preferably 1 to 3%, 1.6 to 2.5% or 1.8 to 2%, relative to the total weight of the composition.

In a particularly preferred embodiment, said composition comprises natural or purified colostrum, preferably purified as defined above, chondroitin sulphate, or the sodium salt thereof, and salicin, each in an amount by weight comprised from 1.5 to 3%, more preferably 1.6 to 2.5% or 1.8 to 2%, relative to the total weight of the composition.

In a preferred embodiment, said composition, for example having a total volume of 50 ml, comprises purified bovine colostrum (900 mg), and more preferably further comprises chondroitin sulphate sodium (800 mg).

In a preferred embodiment, said composition, for example having a total volume of 50 ml, comprises:
- purified bovine colostrum (900 mg);
- chondroitin sulphate sodium 800 mg; and
- salicin 800 mg.

The composition for use according to the present invention can further contain other components commonly used in formulations for intravesical administration, such as, for example, pH buffers, preservatives, diluents and pharmacologically acceptable excipients, in addition to sterile water. Use is preferably made of an apyrogenic sodium chloride and/or disodium hydrogen phosphate anhydrous - Na₂HPO₄, and/or sodium dihydrogen phosphate dihydrate -NaH₂PO₄x2H₂O, and/or water for injectable preparations.

The composition for use according to the present invention preferably has a pH comprised from 6.5 to 8.5, more preferably 7.0 to 8.0 or 7.2 to 7.5.

In a preferred embodiment, the composition for use according to the present invention is in liquid form. Alternatively, the composition for use of the present invention can be in solid form, for example in powder or as a lyophilisate, ready to be dissolved in a physiologically acceptable liquid prior to administration. Advantageously, the composition of the present invention in liquid form is clear and has shown excellent stability. At room temperature and under conditions of accelerated stability no formation of precipitate or turbidity was observed for at least two months, for example for 6-12 months. Moreover, the analyses have shown that there is no trace of any significant degradation of the active ingredients during the preparation and storage of the composition of the present invention.

The inventors have found it useful and extremely advantageous to devise a composition for intravesical administration which comprises an effective amount of colostrum as defined above, and, optionally, chondroitin sulphate and/or salicin.

Said composition can be easily administered to, or self-administered by a subject affected by pelvic pain, acute pelvic pain or chronic pelvic pain, preferably affected by cystitis or interstitial cystitis, by vesical instillation.

The composition can be administered for a limited time, or the administration thereof can be continued over time, for example for several days or 1-2 months, until the disappearance of every manifestation of a pathological condition.

The present invention relates to a device suitable for intravesical administration comprising at least one container which contains the composition comprising colostrum having the characteristics defined above. In the context of the present invention, the term "medical device" is used with the meaning according to Italian Legislative Decree no. 46 of 24 February 1997 and Directive 93/42/EEC of 14 June 1993, i.e. it indicates a substance or another product, whether used alone or in combination, intended by the manufacturer to be used for human beings for the purpose of diagnosis, prevention, monitoring, treatment or alleviation of disease, and which does not achieve its principal intended action in or on the human body for which it is intended by pharmacological, immunological or metabolic means, but which may be assisted in its function by such means.

In said device according to the invention, said composition comprising colostrum is preferably contained in a pre-filled syringe.

Said device contains a volume preferably comprised from 10 to 100 ml, more preferably 20 to 75 ml or 30 to 50 ml of said composition in liquid form.

The following experimental part is provided to illustrate an embodiment of the present invention, without intending to limit the subject matter thereof.

### Experimental part

The protocol of the clinical study was devised for the purpose of evaluating a medical device that comprises the composition of the present invention, containing colostrum, in addition to chondroitin sulphate, salicin, water and phosphate buffer in the treatment of interstitial cystitis.

First of all, it was demonstrated that the composition is suitable for topical (intravesical) administration for human use, as it passed cytotoxicity and biocompatibility tests including mucosal irritation and sensitisation tests on animals.

### COMPOSITION 1

The liquid composition that was the subject of the study was obtained by dissolving the following components in solid form in sterile water (50 ml):
- purified bovine colostrum (900 mg) containing 85-95% by weight of proteins, less than 2% by weight of casein, less than 2% by weight of fats, less than 1.5% by weight of lactose, less than 5% by weight of water;
- phosphate buffer (monosodium and disodium phosphate) until obtaining pH = 7.00.

### COMPOSITION 2

The liquid composition that was the subject of the study was obtained by dissolving the following components in solid form in sterile water (50 ml):
- purified bovine colostrum (900 mg) containing 85-95% by weight of proteins, less than 2% by weight of casein, less than 2% by weight of fats, less than 1.5% by weight of lactose, less than 5% by weight of water;
- chondroitin sulphate (800 mg);

A phosphate buffer (monosodium and disodium phosphate) was also added until obtaining pH = 7.00.

### COMPOSITION 3

The liquid composition that was the subject of the study was obtained by dissolving the following components in solid form in sterile water (50 ml):
- purified bovine colostrum (900 mg) containing 85-95% by weight of proteins, less than 2% by weight of casein, less than 2% by weight of fats, less than 1.5% by weight of lactose, less than 5% by weight of water;
- chondroitin sulphate (800 mg);
- willow extract with a 90% concentration by weight of salicin (corresponding to 800 mg of salicin).

A phosphate buffer (monosodium and disodium phosphate) was also added until obtaining pH = 7.00.

Ten subjects with a diagnosis of interstitial cystitis were enrolled; they had previously been treated with preparations based on chondroitin sulphate and hyaluronic acid without obtaining the desired reduction in symptoms (subjectively assessed by VAS, i.e. visual analogue scale of pain, based on the standard procedure). Composition 1 was administered to these 10 subjects.

Administration took place intravesically, when applicable by self-administration following training and self-catheterisation, twice a week, maintaining the composition in the bladder for about 80 minutes and rotating the body in a lying position, in various suitable positions.

The patients completed a subjective score-based assessment questionnaire (VAS) comprising different parameters correlated to pelvic pain; for each parameter, the subjects had a value from 1 to 10, considering 1 as the lowest pain score (or absence of pain) and 10 as the maximum score (situation at the start of the test).

In view of the positive results of the VAS (values 5-6) after the first two months, administration was continued for another two months, once a week, and a further 1-2 point decrease in the score was achieved until reaching a minimum value dependent on the subjects.

It was found that, advantageously, the composition of the present invention considerably reduces the symptoms of interstitial cystitis also in subjects who had shown to be resistant to prior art therapies based on intravesical administration of GAGs.

## Claims

1. A composition comprising a mixture which comprises or, alternatively, consists of an effective amount of colostrum and at least one pharmaceutical grade additive, said composition being for use in the curative or preventive treatment of symptoms or pathologies of the urinary system, wherein said composition is administered by intravesical administration.

2. The composition for use according to claim 1, wherein said composition is for use in the treatment of pelvic pain, acute pelvic pain or chronic pelvic pain, preferably of cystitis or interstitial cystitis.

3. The composition for use according to either claim 1 or 2, wherein the pathology is in subjects who have been treated by means of radiotherapy or chemotherapy.

4. The composition for use according to any one of the preceding claims, wherein said composition is for use in patients who are resistant to other therapies for chronic pelvic pain, particularly to therapies including the administration of glycosaminoglycans .

5. The composition for use according to any one of the preceding claims, wherein said mixture further comprises at least one compound selected from chondroitin sulphate, or the salts thereof, salicin, and mixtures thereof.

6. The composition for use according to any one of the preceding claims comprising a mixture which comprises or, alternatively, consists of the following components:
a) colostrum;
b) chondroitin sulphate, or the salts thereof, and, optionally,
c) salicin,
said composition further comprising at least one pharmaceutical grade additive.

7. The composition for use according to any one of the preceding claims, wherein the colostrum is present in an amount by weight comprised from 0.5 to 5%, relative to the total weight of the composition and, if present, the chondroitin sulphate, or the salts thereof, and/or salicin are, each independently of the other, in an amount of 0.5 to 5% by weight relative to the total weight of the composition.

8. The composition for use according to claim 7 wherein said mixture comprises or, alternatively, consists of:
- purified bovine colostrum 900 mg;
- chondroitin sulphate sodium 800 mg;
- salicin 800 mg; and
at least one pharmaceutical grade additive.

9. A composition for use according to any one of the proceeding claims which is contained in at least one container comprised in a device suitable for intravesical administration.

## Patentansprüche

1. Zusammensetzung umfassend eine Mischung, umfassend, oder alternativ bestehend aus, einer wirksamen Menge Kolostrum und mindestens ein Additiv pharmazeutischer Qualität, wobei die Zusammensetzung zur Verwendung bei der kurativen oder vorbeugenden Behandlung von Symptomen oder Erkrankungen der Harnwege ist, wobei die Zusammensetzung durch intravesikale Verabreichung verabreicht wird.

2. Zusammensetzung zur Verwendung gemäß Anspruch 1, wobei die Zusammensetzung zur Verwendung in der Behandlung von Beckenschmerzen, akuten Beckenschmerzen oder chronischen Beckenschmerzen, vorzugsweise von Zystitis oder interstitieller Zystitis ist.

3. Zusammensetzung zur Verwendung gemäß Anspruch 1 oder 2, wobei die Erkrankung in Probanden auftritt, die mittels Strahlentherapie oder Chemotherapie behandelt wurden.

4. Zusammensetzung zur Verwendung gemäß mindestens einem der vorhergehenden Ansprüche, wobei die Zusammensetzung zur Verwendung bei Patienten ist, die gegen andere Therapien für chronische Beckenschmerzen, insbesondere Therapien die die Verabreichung von Glykosaminoglykane einschließen, resistent sind.

5. Zusammensetzung zur Verwendung gemäß mindestens einem der vorhergehenden Ansprüche, wobei die Mischung ferner mindestens eine Verbindung umfasst, ausgewählt aus Chondroitinsulfat oder die Salze davon, Salicin, und Mischungen davon.

6. Zusammensetzung zur Verwendung gemäß mindestens einem der vorhergehenden Ansprüche, umfassend eine Mischung umfassend, oder alternativ bestehend aus, den folgenden Komponenten:
a) Kolostrum;
b) Chondroitinsulfat, oder die Salze davon, und optional,
c) Salicin,
die Zusammensetzung ferner umfassend mindestens ein Additiv pharmazeutischer Qualität.

7. Zusammensetzung zur Verwendung gemäß mindestens einem der vorhergehenden Ansprüche, wobei das Kolostrum in einer Gewichtsmenge umfassend von 0,5 bis 5%, bezogen auf das Gesamtgewicht der Zusammensetzung, vorhanden ist, und, wenn vorhanden, das Chondroitinsulfat oder die Salze davon, und/oder Salicin, jeweils unabhängig voneinander in einer Menge von 0,5 bis 5 Gew.-%, bezogen auf das Gesamtgewicht der Zusammensetzung, vorhanden sind.

8. Zusammensetzung zur Verwendung gemäß Anspruch 7, wobei die Mischung umfasst, oder alternativ besteht aus:
- gereinigtem Rinderkolostrum 900 mg;
- Chondroitinsulfat-Natrium 800 mg;
- Salicin 800 mg; und
- mindestens ein Additiv pharmazeutischer Qualität.

9. Zusammensetzung zur Verwendung gemäß mindestens einem der vorhergehenden Ansprüche, die in mindestens einem Behälter enthalten ist, der in einer für die intravesikale Verabreichung geeigneten Vorrichtung umfasst ist.

## Revendications

1. Composition comprenant un mélange qui comprend ou, en variante, est constitué d'une quantité efficace de colostrum et au moins un additif de qualité pharmaceutique, ladite composition étant destinée à être utilisée dans le traitement curatif ou préventif de symptômes ou de pathologies du système urinaire, dans laquelle ladite composition est administrée par voie intravésicale.

2. Composition pour une utilisation selon la revendication 1, dans laquelle ladite composition est destinée à être utilisée dans le traitement d'une douleur pelvienne, d'une douleur pelvienne aiguë ou d'une douleur pelvienne chronique, de préférence d'une cystite ou d'une cystite interstitielle.

3. Composition pour une utilisation selon l'une ou l'autre des revendications 1 et 2, dans laquelle la pathologie est chez des sujets qui ont été traités par radiothérapie ou chimiothérapie.

4. Composition pour une utilisation selon l'une quelconque des revendications précédentes, dans laquelle ladite composition est destinée à être utilisée chez des patients qui sont résistants à d'autres traitements d'une douleur pelvienne chronique, en particulier à des traitements incluant l'administration de glycosaminoglycanes.

5. Composition pour une utilisation selon l'une quelconque des revendications précédentes, dans laquelle ledit mélange comprend en outre au moins un composé choisi parmi le sulfate de chondroïtine, ou ses sels, la salicine, et leurs mélanges.

6. Composition pour une utilisation selon l'une quelconque des revendications précédentes, comprenant un mélange qui comprend ou, en variante, est constitué des composants suivants :
a) du colostrum ;
b) du sulfate de chondroïtine, ou ses sels, et éventuellement
c) de la salicine,
ladite composition comprenant en outre au moins un additif de qualité pharmaceutique.

7. Composition pour une utilisation selon l'une quelconque des revendications précédentes, dans laquelle le colostrum est présent en une quantité en poids comprise entre 0,5 et 5 % par rapport au poids total de la composition et, s'ils sont présents, le sulfate de chondroïtine, ou ses sels, et/ou la salicine, chacun indépendamment des autres, le sont en une quantité de 0,5 à 5 % en poids par rapport au poids total de la composition.

8. Composition pour une utilisation selon la revendication 7, dans laquelle ledit mélange comprend ou, en variante, est constitué de :
- 900 mg de colostrum de bovin purifié ;
- 800 mg de sulfate de chondroïtine sodique ;
- 800 mg de salicine ; et
au moins un additif de qualité pharmaceutique.

9. Composition pour une utilisation selon l'une quelconque des revendications précédentes, qui est contenue dans au moins un récipient compris dans un dispositif adapté à une administration par voie intravésicale.
